# EUROPÄISCHE PATENTSCHRIFT

(11) **EP 1 456 158 B1**
(45) Veröffentlichungstag und Bekanntmachung des Hinweises auf die Patenterteilung: **19.09.2007**
(21) Anmeldenummer: 02792876.1
(22) Anmeldetag: 04.12.2002
(51) Int. Cl.: C07C 29/56, C07C 33/02

(54) **VERFAHREN ZUR ISOMERISIERUNG VON ALLYLALKOHOLEN**
METHOD FOR ISOMERIZING ALLYL ALCOHOLS
PROCEDE D'ISOMERISATION D'ALCOOLS ALLYLIQUES

(30) Priorität: 07.12.2001 DE 10160146
(43) Veröffentlichungstag der Anmeldung: 15.09.2004
(73) Patentinhaber: BASF Aktiengesellschaft, 67056 Ludwigshafen (DE)
(72) Erfinder: HAESE, Frank, 67061 Ludwingshafen (DE); EBEL, Klaus, D-68623 Lampertheim (DE); BURKART, Kirsten, D-67069 Ludwigshafen (DE); UNVERRICHT, Signe, D-68169 Mannheim (DE); MÜNSTER, Peter, D-67354 Römerberg (DE)
(86) Internationale Anmeldenummer: PCT/EP2002/013689
(87) Internationale Veröffentlichungsnummer: WO 2003/048091

(56) Entgegenhaltungen:
- EP-A- 0 860 415
- DE-A- 19 707 385
- DE-A- 19 738 083
- DE-A- 19 958 603

## Beschreibung

Die vorliegende Erfindung betrifft ein Verfahren zur Isomerisierung von Edukt-Allylalkoholen zu Produkt-Allylalkoholen in Gegenwart eines Katalysators, dass dadurch gekennzeichnet ist, dass man Edukt-Allylalkohol in halbkontinuierlicher Fahrweise in einem Katalysator enthaltenden geeigneten Reaktor zu Produkt-Allylalkohol umlagert. Insbesondere wird das erfindungsgemäße Verfahren zur Herstellung von Linalool aus Geraniol und/oder Nerol eingesetzt.

Allylalkohole stellen wichtige Zwischenprodukte in der industriellen organischen Produktsynthese dar. Tertiäre Allylalkohole insbesondere dienen zum Beispiel als Zwischenverbindungen bei der Herstellung von Riechstoffen oder auch als Additive in Seifen und Detergentien.

Allylalkohole isomerisieren unter Säurekatalyse. Diese Isomerisierung entspricht einer 1,3-Wanderung der Hydroxylgruppe und einer internen Verschiebung der Doppelbindung, wie in der folgenden Gleichung mit den allgemeinen Formeln I und II dargestellt:

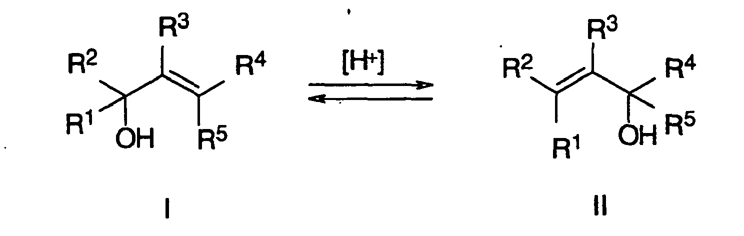

In denen die Reste R¹ bis R⁵ Wasserstoff oder einen ein oder mehrfach ungesättigten oder gesättigten C₁-C₁₂-Alkylrest bedeuten, der gegebenenfalls substituiert sein kann.

Geraniol (2-trans-3,7-Dimethyl-2,6-octadien-8-ol), Nerol (2-cis-3,7-Dimethyl-2,6-octadien-8-ol) und 2-Linalool (3,7-Dimethyl-1,6-octadien-3-ol) sind wichtige Verbindungen in der Riechstoffindustrie. Sie werden sowohl direkt als Riechstoffe eingesetzt oder durch Umsetzung mit anderen Verbindungen zu höher molekularen Riechstoffen umgesetzt. Bedeutung besitzen diese Terpenalkohole auch als C₁₀-Bausteine in der Synthese von Vitaminen, wie dem Vitamin E und dem Vitamin A.

Zunächst wurden die Isomerisierungsreaktionen von Allylalkoholen mit Säuren als Katalysatoren durchgeführt. Diese Verfahren waren jedoch nur von eingeschränkter Bedeutung, da bei ihnen Nebenreaktionen, wie z.B. Dehydratisierungen oder Cyclisierungen dominierten.

Im Stand der Technik werden für die Umlagerung von Edukt-Allylalkoholen zu Produkt-Allylalkoholen, insbesondere für die Umlagerung von Geraniol oder Nerol oder Geraniol/Nerol-Gemischen zu Linalool diskontinuierliche Verfahren beschrieben.

DE 25 16 698 beschreibt ein diskontinuierliches Verfahren zur Umlagerung von Geraniol und/oder Nerol zu Linalool, bei dem das Edukt vorgelegt wird, der Katalysator zugegeben wird, dann das Gleichgewicht eingestellt wird und anschließend das gebildete Linalool aus dem Reaktionsansatz abdestilliert wird.

Nachteilig bei der beschriebenen Verfahrensweise ist, dass die Umlagerung immer wieder unterbrochen werden muß, um im Sumpf anfallende Hochsieder abzutrennen und erneut Katalysator zuzudosieren.

Durch die aufwendige absatzweise diskontinuierliche Reaktionsführung kommt es zu langen Reaktionszeiten sowie für den industriellen Maßstab zu unbefriedigenden Ausbeuten.

Ferner gehen alle im Stand der Technik beschriebenen Verfahren von isoliertem und von Nebenprodukten gereinigtem Edukt-Alyllalkohol aus.

Geht man beispielsweise bei der Herstellung von Linalool direkt von aus der Citral-Hydrierung gewonnenem Nerol und/oder Geraniol aus, so muß man bei der Reaktionsführung auch die in der Citral-Hydrierung anfallenden Nebenprodukte mitberücksichtigen. Dieses Nebenproduktspektrum hat Konsequenzen auf die Umlagerung zum Linalool.

Die Citral-Hydrierung ist diskontinuierlich z.B. in EP 71787 beschrieben.

DE 199 58 603, DE 197 38 083, DE 197 07 385 und EP 0 860 415 offenbaren diskontinuierlich oder kontinuierlich durchgeführte Verfahren zur Isomerisierung von Allylalkoholen.

Aufgabe der vorliegenden Erfindung war es also ein Verfahren zur Isomerisierung von Edukt-Allylalkoholen zu Produkt-Allylalkoholen, insbesondere für die Isomerisierung von Geraniol/Nerol zu Linalool zu entwickeln, daß zum einen nicht die Nachteile des diskontinuierlichen Verfahrens aufweist und zum anderen auch ausgehend von den, nicht von Nebenprodukten gereinigten Edukten zu einem wirtschaftlichen Verfahren mit guten Raum-Zeit-Ausbeuten führt.

Erfindungsgemäß gelöst wurde die Aufgabe durch ein Verfahren zur Isomerisierung von Edukt-Allylalkoholen der allgemeinen Formel (I) zu Produkt-Allylalkoholen der allgemeinen Formel (II) 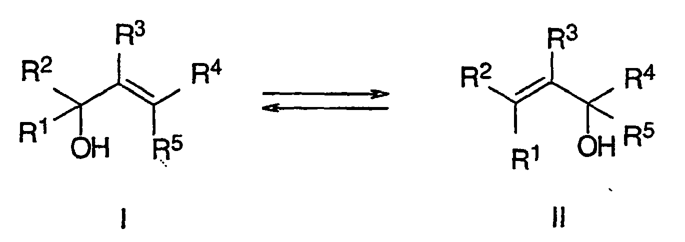 wobei R¹ bis R⁵ Wasserstoff oder einen ein oder mehrfach ungesättigten oder gesättigten C₁-C₁₂-Alkylrest bedeuten, der gegebenenfalls substituiert sein kann,
dadurch gekennzeichnet, dass man mindestens einen Edukt-Allylalkohol oder Gemische davon in halbkontinuierlicher Fahrweise zu dem entsprechenden Produkt-Allylalkohol umlagert, indem man gleichzeitig mindestens einen Edukt-Allylalkohol oder Gemische davon in einen Katalysator enthaltenden geeigneten Reaktor zudosiert.

Bevorzugt wird gleichzeitig der gebildeten Produkt-Allylalkohol abdestilliert.

Als mit Hilfe des erfindungsgemäßen Verfahrens vorteilhaft isomerisierbare Allylalkohole der allgemeinen Formel (I) oder (II) seien beispielsweise genannt: 2-Methyl-3-buten-2-ol, Prenol (3-Methyl-2-buten-1-ol), Linalool, Nerol und Geraniol sowie Farnesol (3,7,11-Trimethyl-dodeca-2,6,10-trien-1-ol) und Nerolidol (3,7,11-Trimethyl-dodeca-1,6,10-trien-3-ol), insbesondere Linalool, Nerol und Geraniol.

Besonders vorteilhaft gestaltet sich das erfindungsgemäße Verfahren, wenn man als Edukt-Allylalkohole Geraniol und/oder Nerol verwendet und als Produkt-Allylalkohol 2-Linalool herstellt.

Die Edukt-Allylalkohole können sowohl ausgehend von einer Vorstufe direkt ohne vorherige Reinigung und Abtrennung der eventuell enthaltenen Nebenprodukte als auch isoliert und gereinigt eingesetzt werden. So liefert beispielsweise die Hydrierung von Citral Geraniol/Nerol-Gemische mit einem spezifischen Nebenproduktspektrum, welches Auswirkungen auf die Umlagerung zum Linalool hat.

Insbesondere folgende Nebenprodukte im Geraniol/Nerol spielen für die Ausführungsform der Umlagerung eine wichtige Rolle:

Citronellol, welches durch Überhydrierung entsteht und je nach Umsatz der Hydrierung in Gehalten von 1 - 10 % im Geraniol/Nerol enthalten sein kann.

Trans - und Cis -3,7-dimethyl-3,5-octadienol die im weiteren Isonerol 1 und Isonerol 2 genannt werden und in Mengen von 0,1 bis 5 % im Hydrieraustrag enthalten sind.

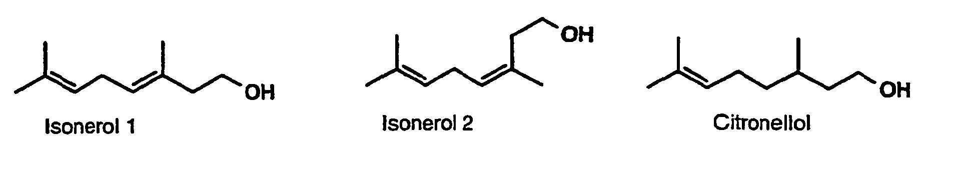

Diese drei Verbindungen lassen sich mit wirtschaftlich vertretbarem Aufwand nicht bzw. nur sehr schwierig von Geraniol und Nerol abtrennen. Deshalb müssen sie mit in die Umlagerungsreaktion geführt werden, sofern man Geraniol und oder Nerol aus der Citral-Hydrierung zu Linalool umlagern möchte.

Als Katalysatoren sind beispielsweise die in der DE 10046865.9 oder die in der WO 0042177 offenbarten geeignet oder auch Dioxowolfram(VI)-Komlexe der allgemeinen Formel (III) 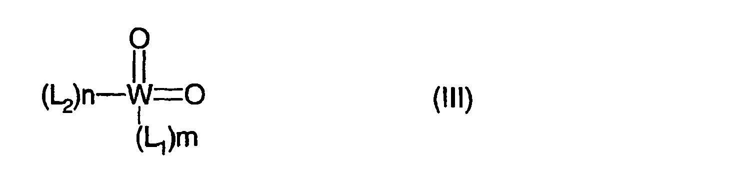 wobei
- L₁, L₂: jeweils unabhängig voneinander einen Liganden ausgewählt aus der Gruppe der Aminoalkohole, der Aminophenole, oder Gemischen davon bedeuten,
- m, n: die Zahl 1 oder 2 bedeutet.

Für die Ausführung der Isomerisierung bestehen drei Verfahrens-varianten. Die Isomerisierung kann entweder in einem Reaktor oder in einer Kaskade aus mindestens 3 Reaktoren oder in einer Reaktivkolonne (Loräreaktor), welche mit sogenannten Reaktorzonen im Mittelraum ausgestattet ist, um die erforderlichen Verweilzeiten zu erreichen, erfolgen.

Wichtig für die Ausführungsform der Umlagerung ist die Tatsache, dass die Umlagerung eine Gleichgewichtsreaktion ist, und das im Falle der Isomerisierung von Geraniol und/oder Nerol das gewünschte Produkt Linalool einen gegenüber Geraniol und Nerol geringeren Siedepunkt besitzt, so dass es destillativ aus dem Gleichgewicht abgetrennt werden kann. Dadurch wird in der Gleichgewichtsreaktion immer wieder neues Linalool nachgebildet.

Im folgenden werden die Verfahrensvarianten in allgemeiner Weise am Beispiel der Isomerisierung von Geraniol und/oder Nerol zu Linalool verdeutlicht.

Führt man das Verfahren in halbkontinuierlicher Fahrweise aus, wird in einem Reaktor mit angeschlossener Destillatonskolonne zunächst Geraniol oder Nerol oder Geraniol/Nerol-Gemisch vorgelegt. Dann wird der Katalysator zudosiert und man beginnt gebildetes Linalool abzudestillieren. Gleichzeitig wird weiteres Geraniol oder Nerol oder Geraniol/Nerol-Gemisch zudosiert um den Stand im Reaktor konstant zu halten. Auch kann während des Prozesses weiterer Katalysator entweder kontinuierlich oder diskontinuierlich zugegeben werden um einer teilweisen Desaktivierung des Katalysators entgegenzuwirken.

Geht man bei den Geraniol und/oder Nerol Gemischen direkt von aus der Citral-Hydrierung gewonnenem Geraniol bzw. Nerol aus, pegeln sich im Verlauf des Semibatch-Ansatzes im Reaktor die Nebenprodukte, die einen höheren Siedepunkt besitzen als Linalool, immer mehr auf. Dadurch steht natürlich ein immer geringerer Anteil des Reaktorvolumens für die gewünschte Reaktion, die Umlagerung von Geraniol bzw. Nerol zu Linalool zur Verfügung, wodurch die Linalool-Bildungsrate im Lauf der Zeit abnimmt.

Wenn die Linalool-Bildungsrate durch die im Reaktor aufgepegelten Nebenprodukte zu stark abnimmt, stellt man die Dosierung von Geraniol oder Nerol oder Geraniol/Nerol-Gemisch ein und destilliert weiter Linalool ab, bis nahezu das gesamte noch im Reaktor enthaltene Geraniol und Nerol zu Linalool umgesetzt ist. Zu diesem Zeitpunkt enthält der Reaktor neben sehr geringen Mengen Linalool, Geraniol und Nerol noch die genannten Nebenprodukte, den Katalysator und während der Reaktion gebildete Hochsieder.

Nun werden die Nebenprodukte Citronellol, Isonerol 1 und Isonerol 2 von den Hochsiedern und dem Katalysator abdestilliert.

Der dabei verbleibende Sumpf kann entweder entsorgt werden, oder falls der darin enthaltene Katalysator noch hinreichend aktiv ist, kann man den Sumpf auch erneut in den nächsten Ansatz wieder einsetzen. Des weiteren kann man den Katalysator z.B. durch Kristallisation vom Sumpf abtrennen und den so zurückgewonnenen Katalysator wieder einsetzten.

So können die Nebenprodukte Citronellol, Isonerol 1 und Isonerol 2 ohne Verluste an Wertprodukten abgetrennt und anschließend isoliert werden und gegebenenfalls einer weiteren Verwertung zugeführt werden.

Wenn man Geraniol oder Nerol oder Geraniol/Nerol-Gemische in die Reaktion einsetzt, welche die genannten Nebenprodukte Citronellol, Isonerol 1 und Isonerol 2 und auch weitere von Geraniol bzw. Nerol schwierig abtrennbare Nebenprodukte nicht oder nur in sehr geringer Menge enthalten, kann der Zeitraum in welchem der Einsatzstoff dosiert und das Linalool abdestilliert wird deutlich verlängert werden, weil sich pro Zeiteinheit weniger Nebenprodukte im Sumpf aufpegeln. Dabei empfiehlt sich eine Nachdosierung von Katalysator, sofern die Aktivität des Katalysators durch Desaktivierung zu stark nachlässt.

Außerdem kann auf die Abtrennung der Nebenprodukte am Ende des Semibatch-Ansatzes verzichtet werden.

Auch ist das Verfahren für die Umlagerung von Geraniol der Nerol, oder Geraniol/Nerolgemischen, welche andere von Geraniol bzw. Nerol schwierig abtrennbare Nebenprodukte enthalten, sehr gut geeignet.

Bei der vollkontinuierlichen Verfahrensvariante wird in einen kontinuierlich betriebenen Reaktor gleichzeitig kontinuierlich Katalysator und Geraniol oder Nerol, oder ein Gemisch von Geraniol und Nerol dosiert und gebildetes Linalool kontinuierlich abdestilliert. Außerdem wird zur Ausschleusung von Hochsiedern ebenfalls kontinuierlich eine anteilige Menge des Reaktorinhalts entnommen und die in diesem Anteil noch enthaltenen Wertprodukte Geraniol, Nerol und Linalool destillativ z.B. über eine einfache Verdampfung oder über eine Kolonne von während der Reaktion gebildeten Hochsiedern abgetrennt. Dabei verbleibt der nicht flüchtige Katalysator entweder in den Hochsiedern welche einer Entsorgung zugeführt werden, oder er kann durch Kühlung aus den Hochsiedern ausgefällt, abgetrennt und wieder erneut eingesetzt werden. Die abdestillierten Wertprodukte werden wieder in den Reaktor zurückgeführt.

Dieses Verfahren ermöglicht eine voll kontinuierliche Umlagerung von Geraniol oder Nerol, oder Gemischen von Geraniol und Nerol zu Linalool, ohne dass die Reaktion zur Ausschleusung von Hochsiedern, oder zur Zugabe des Katalysators unterbrochen werden muss.

Sowohl das kontinuierliche als auch das halbkontinuierliche Verfahren werden bei Temperaturen von 130 - 220°C bevorzugt 150 - 200 °C entweder drucklos, oder im Vakuum durchgeführt. Bei Reaktionstemperaturen, welche deutlich unterhalb des Siedepunkts von Linalool liegen, empfiehlt sich die Durchführung im Vakuum, um das Abdestillieren von Linalool aus dem Reaktor zu ermöglichen.

Die Isomerisierung kann auch in einer Anlage aus mehreren hintereinander geschalteten Reaktoren in Kaskadenschaltung erfolgen. Die Zahl der verwendeten Reaktoren liegt im allgemeinen zwischen 2 und 10, bevorzugt zwischen 2 und 5, insbesonders bevorzugt verwendet man 3 Reaktoren.

In den ersten Reaktor werden gleichzeitig Geraniol und/oder Nerol sowie Katalysator eindosiert und bei einem Druck von 60 bis 1000 mbar und einer Temperatur von 150 bis 200°C teilweise isomerisiert. Diese teilumgesetzte Mischung wird in einen zweiten Reaktor gepumpt in dem in Bezug auf Geraniol und Nerol Vollumsatz erfolgt. Der Vollumsatz im zweiten Reaktor wird ermöglicht durch die Entnahme eines Dampfstromes je aus dem ersten und dem zweiten Reaktor. Beide Dampfströme werden zusammengeführt und in der Kolonne des ersten Reaktors rektifiziert. Als Kopfprodukt werden Linalool, Leichtsieder und Wasser abgetrennt und Geraniol/Nerol in den ersten Reaktor zurückgeführt. Im 3. Reaktor befinden sich Schwersieder und Katalysator sowie gegebenenfalls Citronellol und Isonerol. Citronellol und Isonerol werden gegebenenfalls in der Kolonne des dritten Reaktors gemeinsam als Kopfprodukt abgetrennt. Aus dem Sumpf des dritten Reaktors wird ein kontinuierlicher Flüssigstrom abgezogen und in einem Verdampfer Citronellol gemeinsam mit Isonerolen von Schwersiedern und Katalysator abgetrennt. Schwersieder und Katalysator werden verworfen; Citronellol und Isonerole aus dem Verdampfer werden in den Sumpf des dritten Reaktors zurückgeführt.

Als Reaktoren bei allen Verfahren sind nicht rückvermischte Reaktoren, wie Rohrreaktoren oder mit Einbauten versehene Verweilzeitbehälter, vorzugsweise aber rückvermischte Reaktoren, wie Rührkessel, Schlaufenreaktoren, Strahlschlaufenreaktoren oder Strahldüsenreaktoren geeignet. Es können aber auch Kombinationen aus aufeinander folgenden rückvermischten Reaktoren und nicht rückvermischten Reaktoren verwendet werden.

Gegebenenfalls können auch mehrere Reaktoren in einer mehrstufigen Apparatur zusammengefaßt werden. Solche Reaktoren sind zum Beispiel Schlaufenreaktoren mit eingebauten Siebböden, kaskadierte Behälter, Rohrreaktoren mit Zwischeneinspeisung oder Rührkolonnen. Es empfiehlt sich die einzelnen Reaktoren alle oder teilweise mit Wärmetauschern auszustatten. In den Reaktoren muß für eine gute Durchmischung der Reaktanten gesorgt werden, was beispielsweise durch Rühren oder Umpumpen, gegebenenfalls in Kombination mit dem Behandeln mit statischen Mischern oder Mischdüsen erfolgen kann.

Das Volumen der Reaktoren ist so zu bemessen, daß die mittlere Verweilzeit der Reaktionsmischung in den Reaktoren zwischen 5 Minuten und 8 Stunden, insbesondere zwischen 10 Minuten und 5 Stunden beträgt. Mit besonderem Vorteil arbeitet man mit Reaktoren, die etwa das gleiche Volumen aufweisen, prinzipiell können aber auch Reaktoren mit unterschiedlichen Volumina eingesetzt werden.

Die Temperatur in den Reaktoren liegt im allgemeinen zwischen 150 und 200°C.

Der Druck in den Reaktoren ist nicht kritisch, er sollte aber so hoch sein, daß der Inhalt der Reaktoren nahezu flüssig bleibt. Im allgemeinen sind dazu Drücke von 1 bar bis 40 bar erforderlich, mit Vorteil liegt der Druck zwischen 1 und 6 bar.

In einer weiteren Verfahrensvariante kann die Isomerisierung in einer Destillationskolonne, ausgestattet mit sogenannten Lordreaktoren (durchmischter Flüssigraum mit nur geringem Druckverlust) durchgeführt werden.

Der Lordreaktor zeichnet sich durch mindestens zwei, in Längsrichtung übereinander angeordnete Kammern aus, wobei die Kammern voneinander durch flüssigkeitsdichte Böden getrennt sind, jede Kammer durch je einen Flüssigkeitsüberlauf mit der unmittelbar darunterliegenden Kammer verbunden ist und über den Flüssigkeitsüberlauf der untersten Kammer ein flüssiger Produktstrom abgezogen wird, der Gasraum oberhalb des Flüssigkeitsspiegels in jeder Kammer mit der jeweils unmittelbar darüber angeordneten Kammer durch ein oder mehrere Leitrohre verbunden ist, das (die) jeweils in einen Gasverteiler mit Öffnungen für den Gasaustritt unterhalb des Flüssigkeitsspiegels mündet, sowie mit jeweils mindestens einem um jeden Gasverteiler vertikal angeordnetem Leitblech, dessen oberes Ende unterhalb des Flüssigkeitsspiegels und desssen unteres Ende oberhalb des flüssigkeitsdichten Bodens der Kammer endet und das jede Kammer in einen oder mehrere begaste und in einen oder mehrere unbegaste Räume trennt.

Die Katalysatorlösung wird zwischen oberem Trennteil und darunterliegender Reaktionszone, die aus mehreren untereinanderliegenden Lordreaktoren besteht, auf einen Kolonnenboden gegeben.

Oberhalb der Katalysatoreindosierstelle befindet sich der Trennteil, innerhalb dessen eine Auftrennung des Stromes in Linalool, Leichtsieder und Wasser sowie in Geraniol, Nerol und gegebenenfalls Citronellol und Isonerole. Unterhalb der Katalysatoreindosierstelle fließt der Katalysator in die Reaktionszonen. Im untersten Reaktionsraum, also kurz oberhalb des Sumpfes soll annähernd alles Geraniol/Nerol durch Isomerisierung verbraucht sein. Hier sollen durch Entnahme eines Seitenstromes die gegebenenfalls vorhandenen Citronellole und Isonerole teilweise abgetrennt werden. Der Sumpf-Inhalt wird zu einem bestimmten Anteil kontinuierlich durch einen Verdampfer geführt. Die nicht verdampfbaren Hochsieder mit Katalysator aus dem Sumpf werden teilweise verworfen und teilweise zum Frischgeraniol/Nerol zurückgeführt. Die verdampfbaren Sumpf-Bestandteile aus dem Verdampfer bestehen überwiegend, sofern vorhanden aus Citronellol und Isonerolen und werden in den Sumpf zurückgeführt.

Die folgenden Beispiele sollen das erfindungsgemäße Verfahren näher beschreiben ohne es jedoch darauf einzuschränken:

### Kontinuierliches Verfahren

### Vergleichsbeispiel 1

In einem 1,6 l-Rührreaktor mit aufgesetzter Destillationskolonne, werden 825 g Geraniol/Nerol-Gemisch mit einem Gehalt von 69,5 % Geraniol und 29,5 % Nerol vorgelegt und auf 160°C aufgeheizt und ein Vakuum von 132 - 135 mbar angelegt. Dann wird eine Mischung aus 5,14 g einer Wolframoxodiperoxo-Lösung (hergestellt durch Auflösen von 1,29 g Wolframsäure in 3,86 g 30%igem Wasserstoffperoxid über 6 h bei 40°C) und einer Lösung von 2,63 g 8-Hydroxychinolin in 26,3 g Methanol zugegeben. Gleichzeitig beginnt man über die Destillationskolonne zunächst die Lösungsmittel des Katalysators und anschließend gebildetes Linalool abzudestillieren. Dazu wird die über Kopf genommene Linalool-Menge über einen Rücklaufteiler durch die Kopftemperatur der Kolonne geregelt. Dabei werden bei einer Kopftemperatur von 133,8°C kontinuierlich 110 g Linalool pro Stunde mit einem Gehalt von etwa 98 % Linalool abdestilliert. Gleichzeitig werden standgeregelt ebenfalls kontinuierlich etwa die gleiche Menge (112 g/h) Geraniol/Nerol-Gemisch und 1 g/h eines Gemischs der oben beschriebenen Lösungen von Wolframsäure in 30 %igem Wasserstoffperoxid und von 8-Hydroxychinolin in Methanol zudosiert. Des weiteren werden 110 g/h der Reaktionsmischung ebenfalls kontinuierlich über einen Dünnschichtverdampfer von Hochsiedern befreit. Der Kopfaustrag des Dünnschichtverdampfers 107 g/h besteht im Wesentlichen aus Geraniol, Nerol und Linalool und wird wieder in den Reaktor zurückgeführt. Der Sumpfablauf des Dünnschichtverdampfers (3 g/h) enthält noch geringe Mengen Geraniol, Nerol und Linalool sowie die im Reaktor gebildeten Hochsieder und einen Teil des Katalysators und wird verworfen.

### Vergleichsbeispiele 2 und 3

Die Reaktion wurde völlig analog mit reinem Geraniol und auch mit reinem Nerol als Einsatzstoff durchgeführt, wobei identische Ergebnisse wie mit dem Geraniol/Nerol-Gemisch als Einsatzstoff erhalten wurden.

### Halbkontinuierliches Verfahren

### Beispiel 4

In einem 1,6 l -Rührreaktor mit aufgesetzter Destillationskolonne, wurden 800 g Geraniol/Nerol-Gemisch aus der diskontinuierlichen Hydrierung von Citral mit einem Gehalt von 72 % Geraniol, 21 % Nerol, 2 % Citronellol, 2 % Isonerol 1 und 1 % Isonerol 2 vorgelegt, auf 160°C aufgeheizt und ein Vakuum von 132 - 135 mbar angelegt. Dann wurde eine Mischung aus 5,14 g einer Wolframoxodiperox-Lösung (hergestellt durch Auflösen von 1,29 g Wolframsäure in 3,86 g 30%igem Wasserstoffperoxid über 6 h bei 40°C) und einer Lösung von 2,63 g 8-Hydroxychinolin in 26,3 g Methanol zugegeben. Gleichzeitig begann man über die Destillationskolonne zunächst die Lösungsmittel des Katalysators und anschließend gebildetes Linalool abzudestillieren. Dazu wurde die über Kopf genommene Linalool Menge über einen Rücklaufteiler durch die Kopftemperatur der Kolonne, die in diesem Versuch auf 133,8°C gehalten wurde, geregelt. Gleichzeitig wurden standgeregelt ebenfalls kontinuierlich etwa so viel Geraniol/Nerol-Gemisch der obengenannten Zusammensetzung zudosiert, wie Linalool abdestilliert wurde. Die Versuchsdauer betrug bis zur Einstellung der Dosierung 79 h. Dabei wurden zusätzlich zum vorgelegten Geraniol/Nerol-Gemisch kontinuierlich noch weitere 6305 g Geraniol-Nerol-Gemisch und nach 30 h Versuchsdauer einmalig nochmals die gleiche Menge Katalysatorlösung wie oben beschrieben zudosiert..Nach dem Abstellen der Geraniol/Nerol-Dosierung wurde noch so lange weiter abdestilliert, bis kein Linalool, mehr über Kopf ging. Die insgesamt abdestillierte Linalool-Menge beträgt 6570 g 98 %iges Linalool. Dann wurde das Vakuum auf 120 - 80 mbar abgesenkt und 289 g eines Gemischs abdestilliert welches 41 % Citronellol, 28 % Isonerol 1, 18 % Isonerol 2 und 4 % Linalool enthielt. Nach dieser Destillation verblieben 177 g Sumpf, welcher verworfen wurde.

### Beispiele 5-8

Die Umlagerung aus Beispiel 1 wurde analog mit folgenden Einsatzstoffen durchgeführt:

### Beispiel 5

Gemisch aus 45 % Geraniol, 50 % Nerol, 4 % Citronellol, 0,6 % Isonerol 1 und 2

### Beispiel 6

Gemisch aus 96 % Geraniol, 0,2 % Nerol, 0,1 % Citronellol, 3,9 % Isonerol 1 und 2

### Beispiel 7

99 %iges Geraniol

### Beispiel 8

99 %iges Nerol

Bei den Beispielen 7 und 8 konnte der Zeitraum in welchem der Einsatzstoff dosiert und das Linalool abdestilliert wurde gegenüber Beispiel 1 verdoppelt werden, weil sich pro Zeiteinheit weniger Nebenprodukte im Sumpf aufpegelten. Dabei wurde nach 80 h und nach 115 h nochmals Katalysator nachdosiert. Außerdem wurde auf die Abtrennung der Nebenprodukte am Ende des Semibatch-Ansatzes verzichtet.

## Patentansprüche

1. Verfahren zur Isomerisierung von Edukt-Allylalkoholen der allgemeinen Formel (I) zu Produkt-Allylalkoholen der allgemeinen Formel (II) 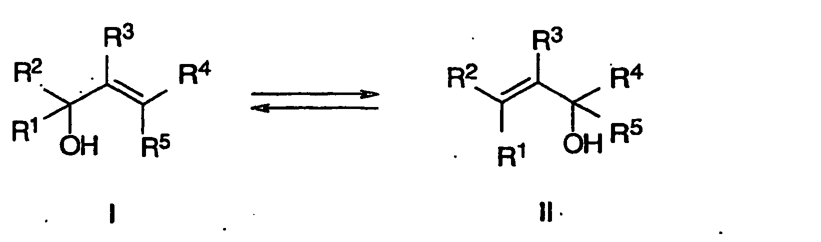 wobei R1 bis R5 Wasserstoff oder einen ein oder mehrfach ungesättigten oder gesättigten C₁-C₁₂-Alkylrest bedeuten, der gegebenenfalls substituiert sein kann,
**dadurch gekennzeichnet, dass** man mindestens einen Edukt-Allylalkohol oder Gemische davon in halbkontinuierlicher Fahrweise zu dem entsprechenden Produkt-Allylalkohol umlagert, indem man gleichzeitig mindestens einen Edukt-Allylalkohol oder Gemische davon in einen Katalysator enthaltenden geeigneten Reaktor zudosiert.

2. Verfahren gemäß Anspruch 1, **dadurch gekennzeichnet, dass** gleichzeitig der gebildete Produkt-Allylalkohol abdestilliert wird.

3. Verfahren gemäß Anspruch 1 und 2, **dadurch gekennzeichnet, dass** als Edukt-Allylalkohol Geraniol, Nerol oder ein Gemisch davon eingesetzt wird.

4. Verfahren gemäß Anspruch 3, **dadurch gekennzeichnet, dass** das Geraniol und/oder Nerol direkt aus der Citralhydrierung eingesetzt werden.

5. Verfahren gemäß einem der Ansprüche 3 und 4, **dadurch gekennzeichnet, dass** das Geraniol/Nerol einen Gehalt an Citronellol von 1 bis 10 % aufweist.

6. Verfahren gemäß einem der Ansprüche 3 bis 5, **dadurch gekennzeichnet, dass** das Geraniol/Nerol einen Gehalt an Trans- und Cis-3,7-dimethyl-3,5-octadienol von 0,1 bis 5 % aufweist.

7. Verfahren gemäß einem der Ansprüche 1 bis 6, **dadurch gekennzeichnet, dass** man als Katalysator einen oder ein Gemisch von verschiedenen Dioxowolfram(VI)-Komplex der allgemeinen Formel III 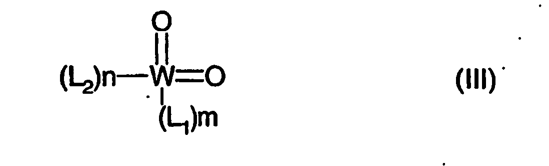 wobei
L₁, L₂ jeweils unabhängig voneinander einen Liganden ausgewählt aus der Gruppe der Aminoalkohole, der Aminophenole oder Gemischen davon bedeuten und
m, n die Zahl 1 oder 2 bedeutet
einsetzt.

8. Verfahren gemäß einem der Ansprüche 1 bis 7, **dadurch gekennzeichnet, dass** die Isomerisierung in einem Rührkessel stattfindet.

9. Verfahren gemäß einem der Ansprüche 1 bis 8, **dadurch gekennzeichnet, dass** die Isomerisierung in einer Kaskade aus Reaktoren stattfindet.

10. Verfahren gemäß einem der Ansprüche 1 bis 9, **dadurch gekennzeichnet, dass** die Isomerisierung in einer Reaktivkolonne (Lordreaktor) stattfindet.

11. Verfahren gemäß einem der Ansprüche 1 bis 10, **dadurch gekennzeichnet, dass** man das Verfahren bei Temperaturen von 130 bis 220°C durchführt.

12. Verfahren gemäß einem der Ansprüche 1 bis 11, **dadurch gekennzeichnet, dass** man den in den abgetrennten Nebenprodukten enthaltenen Katalysator isoliert und gegebenenfalls wieder einsetzt.

## Claims

1. A process for isomerizing reactant allyl alcohols of the general formula (I) to product allyl alcohols of the general formula (II) 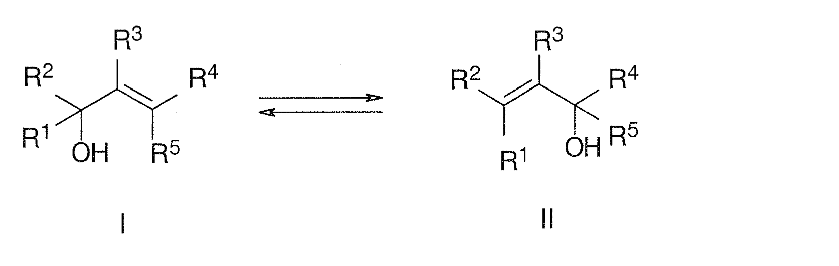 where R1 to R5 are each hydrogen or a mono- or polyunsaturated or saturated C₁-C₁₂-alkyl which may optionally be substituted,
wherein at least one reactant allyl alcohol or mixtures thereof is rearranged in semicontinuous mode to give the corresponding product allyl alcohol, by simultaneously metering in at least one reactant allyl alcohol or mixtures thereof into a catalyst-comprising suitable reactor.

2. The process according to claim 1, wherein the product allyl alcohol formed is simultaneously distilled off.

3. The process according to either of claims 1 and 2, wherein the reactant allyl alcohol used is geraniol, nerol or a mixture thereof.

4. The process according to claim 3, wherein the geraniol and/or nerol are used directly from the citral hydrogenation.

5. The process according to either of claims 3 and 4, wherein the geraniol/nerol has a citronellol content of from 1 to 10%.

6. The process according to any of claims 3 to 5, wherein the geranial/nerol has a trans- and cis-3,7-dimethyl-3,5-octadienol content of from 0.1 to 5%.

7. The process according to any of claims 1 to 6, wherein the catalyst used is one dioxotungsten (VI) complex, or a mixture of different dioxotungsten (VI) complexes, of the general formula III 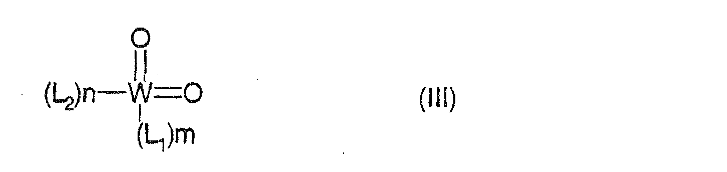 where
L₁, L₂ are each independently a ligand selected from the group of the amino alcohols, the aminophenols and mixtures thereof, and
m, n are each the number 1 or 2.

8. The process according to any of claims 1 to 7, wherein the isomerization takes place in a stirred tank.

9. The process according to any of claims 1 to 8, wherein the isomerization takes place in a battery of reactors.

10. The process according to any of claims 1 to 9, wherein the isomerization takes place in a reactive column (Lord reactor).

11. The process according to any of claims 1 to 10, which is carried out at temperatures of from 130 to 220°C.

12. The process according to any of claims 1 to 11, wherein the catalyst present in the by-products removed is isolated and if appropriate reused.

## Revendications

1. Procédé d'isomérisation d'alcools allyliques de départ de la formule générale (I) en alcools allyliques de produit de la formule générale (II) : 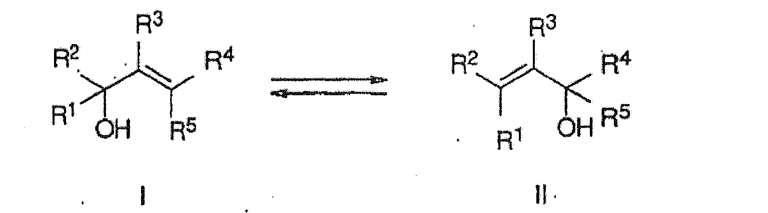 où R¹ à R⁵ représentent de l'hydrogène ou un radical alkyle en C₁-C₁₂ saturé ou insaturé à une ou à plusieurs reprises, qui peut éventuellement être substitué,
**caractérisé en ce qu'**on transpose au moins un alcool allylique de départ ou des mélanges de ceux-ci selon un mode opératoire semi-continu en l'alcool allylique de produit correspondant, en ajoutant de manière dosée simultanément au moins un alcool allylique de départ ou des mélanges de ceux-ci dans un réacteur approprié contenant un catalyseur.

2. Procédé suivant la revendication 1, **caractérisé en ce que** simultanément l'alcool allylique de produit formé est séparé par distillation.

3. Procédé suivant la revendication 1 et 2, **caractérisé en ce que**, comme alcool allylique de départ, on met en ouvre du géraniol, du nérol ou un mélange de ceux-ci.

4. Procédé suivant la revendication 3, **caractérisé en ce que** le géraniol et/ou le nérol sont mis en oeuvre directement en provenance de l'hydrogénation de citral.

5. Procédé suivant l'une des revendications 3 et 4, **caractérisé en ce que** le géraniol/nérol présente une teneur en citronellol de 1 à 10 %.

6. Procédé suivant l'une des revendications 3 à 5, **caractérisé en ce que** le géraniol/nérol présente une teneur en trans- et cis-3,7-diméthyl-3,5-octadiénol de 0,1 à 5 %.

7. Procédé suivant l'une des revendications 1 à 6, **caractérisé en ce que**, comme catalyseur, on met en oeuvre un complexe de dioxotungstène(VI) ou un mélange de différents complexes de dioxotungstène(VI) de la formule générale (III) : 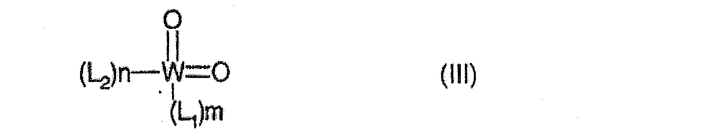 dans laquelle
L₁, L₂ représentent chacun, indépendamment l'un de l'autre, un ligand choisi parmi le groupe des aminoalcools, des aminophénols ou de leurs mélanges, et
m, m représentent le nombre 1 ou 2.

8. Procédé suivant l'une des revendications 1 à 7, **caractérisé en ce que** l'isomérisation a lieu dans une cuve à agitation.

9. Procédé suivant l'une des revendications 1 à 8, **caractérisé en ce que** l'isomérisation a lieu dans une cascade de réacteurs.

10. Procédé suivant l'une des revendications 1 à 9, **caractérisé en ce que** l'isomérisation a lieu dans une colonne réactive (Lordreactor).

11. Procédé suivant l'une des revendications 1 à 10, **caractérisé en ce qu'**on effectue le procédé à des températures de 130 à 220°C.

12. Procédé suivant l'une des revendications 1 à 11, **caractérisé en ce qu'**on isole le catalyseur contenu dans les produits secondaires séparés et éventuellement on le remet en oeuvre.
